# EUROPEAN PATENT SPECIFICATION

(11) **EP 2 167 664 B1**
(45) Date of publication and mention of the grant of the patent: **25.01.2012**
(21) Application number: 08774584.0
(22) Date of filing: 01.07.2008
(51) Int. Cl.: C12N 15/70, A61K 39/02

(54) **HYBRID OPERON FOR EXPRESSION OF COLONIZATION FACTOR (CF) ANTIGENS OF ENTEROTOXIGENIC ESCHERICHIA COLI**
HYBRIDOPERON ZUR EXPRESSION VON KOLONISATIONSFAKTORANTIGENEN ENTEROTOXIGENER ESCHERICHIA COLI
OPÉRON HYBRIDE POUR L'EXPRESSION D'ANTIGÈNES DU FACTEUR DE COLONISATION (CF) D'ESCHERICHIA COLI ENTÉROTOXINOGÈNE

(30) Priority: 02.07.2007 EP 07111501
(43) Date of publication of application: 31.03.2010
(73) Proprietor: Gotovax AB, 426 74 Västra Frölunda (SE)
(72) Inventor: LEBENS, Michael, S-523 99 Hökerum (SE); SVENNERHOLM, Ann-Mari, S-426 74 Västra Frölunda (SE); TOBIAS, Joshua, S-415 10 Göteborg (SE)
(74) Representative: Linde, Jörgen
(86) International application number: PCT/EP2008/058438
(87) International publication number: WO 2009/004002

(56) References cited:
- EP-A- 0 060 129
- EP-A- 1 543 836
- WO-A-03/022306
- GIRON J A ET AL: "Simultaneous expression of CFA/I and CS3 colonization factor antigens of enterotoxigenic Escherichia coli by DELTAaroC, DELTAaroD Salmonella typhi vaccine strain CVD 908" VACCINE, BUTTERWORTH SCIENTIFIC. GUILDFORD, GB, vol. 13, no. 10, 1 July 1995 (1995-07-01), pages 939-946, XP004057513 ISSN: 0264-410X
- BARRY EILEEN M ET AL: "Immune responses elicited against multiple enterotoxigenic Escherichia coli fimbriae and mutant LT expressed in attenuated Shigella vaccine strains." VACCINE 17 JAN 2003, vol. 21, no. 5-6, 17 January 2003 (2003-01-17), pages 333-340, XP002480907 ISSN: 0264-410X
- GAASTRA WIM ET AL: "Antigenic variation within the subunit protein of members of the colonization factor antigen I group of fimbrial proteins in human enterotoxigenic Escherichia coli." INTERNATIONAL JOURNAL OF MEDICAL MICROBIOLOGY : IJMM JUN 2002, vol. 292, no. 1, June 2002 (2002-06), pages 43-50, XP002480908 ISSN: 1438-4221

## Description

The present invention relates to hybrid operons for expression of colonization factor (CF) antigens of enterotoxigenic *Escherichia coli,* and in particular to a recombinant operon comprising genes including at least two structural genes for expression of two different CFs. The invention further relates to host cells comprising such an operon in the genom of the cell or in a plasmid inserted into the cell, such as an *Escherichia coli* cell.

### Background of the Invention

Enterotoxigenic *Escherichia coli* (ETEC) is a major cause of travelers diarrhea and of diarrheal morbidity and mortality of children in endemic areas in many parts of the world. Virulence of the bacteria is associated with expression of fimbrial colonization factors (CFs) (Gaastra and Svennerholm, 1996) which mediate bacterial adhesion to the intestine and with secretion of heat-labile (LT) and/or heat-stable (ST) toxins which by affecting electrolyte and fluid transport processes in the gut are responsible for the diarrhea characteristic of the disease (Qadri *et al,* 2005a; Sanchez and Holmgren, 2005).

Protection against ETEC disease is associated with antibody-mediated neutralization of LT and immune responses against the CFs (Levine *et al,* 1994, Svennerholm and Holmgren, 1995; Svennerholm and Savarino, 2004). In general, the purpose of a vaccine is to induce an immune response in recipients that provides protection against subsequent challenge with the actual pathogen. This may be achieved by inoculation with a live attenuated strain of a pathogen, i.e. a strain having reduced virulence such that it does not cause the disease while still stimulating an effective immune response, or by administration of one or more killed strains of the pathogen that can elicit protective immune responses that are effective against infecting virulent strains. For immunization against enteric infections the vaccine should preferably be given by the oral route to efficiently stimulate an effective immune response locally in the intestinal mucosa, but also other mucosal routes or parenteral or even transcutanous routes may be used for inducing protective immunity.

Development of an effective vaccine that protects against disease caused by ETEC is difficult. More than 100 different serotypes have been associated with pathogenic strains. Furthermore, these strains can carry one or more of a large number of CFs (each of which is antigenically different) that facilitate the establishment of the infection in the intestine (Qadri *et al,* 2005a).

There is considerable evidence that immune responses directed against the CFs are protective, and that mucosal immune responses in the intestine are of particular importance for protection (Svennerholm *et al,* 1988, 1990; Levine *et al,* 1994; Svennerholm and Savarino, 2004). To induce such responses an ETEC vaccine should preferably be administered orally. We have previously developed an oral killed whole cell ETEC vaccine, containing five strains representing common ETEC serotypes and expressing several of the most commonly encountered CFs (in several cases usually referred to as coli surface [CS] proteins), i.e. CFA/I, CS1, CS2, CS3, CS4, and CS5 together with recombinant cholera toxin B subunit (CTB, which is highly homologous to the B subunit of ETEC LT) (Svennerholm and Holmgren, 1995; Svennerholm and Savarino, 2004). Initial clinical trials with this vaccine gave rise to significant immune responses against both CTB and the specific CFs present in the vaccine in Swedish volunteers and subsequently in adults and children in Egypt and Bangladesh (Jertborn *et al,* 1993; Ahren *et al,* 1998; Savarino *et al,* 1998, 1999, Qadri *et al,* 2005a, 2005b). The vaccine also provided significant protection against diarrhea sufficiently severe to interfere with the daily activity of American travelers going to Mexico and Guatemala (Sack *et al,* 2002; Svennerholm and Savarino 2004). However, the protection efficacy of the vaccine in Egyptian infants, 6-18 months of age was found to be low (Savarino et al, to be published). This suggested that whereas the vaccine was effective against more severe disease in travelers, it was not sufficiently potent to protect infants living in endemic areas (Svennerholm and Steele, 2004)

One of the reasons for the low efficacy of the described ETEC vaccine in infants is thought to be due to the comparatively low antibody responses found to the CF antigens in this age group (Savarino and Svennerholm, 2004). This poor response may be improved by giving higher dose of the different CFs, and hence increasing the amount of these antigens in a vaccine dose is a priority for continued development of a killed ETEC whole-cell vaccine. It is not feasible simply to increase the number of ETEC bacteria administered with each vaccine dose since it has been shown that giving high amounts of inactivated *E*. *coli* bacteria (even of an *E. coli* K12 placebo preparation) to young children 6-18 months of age can result in adverse reactions in the form of vomiting, probably due to the large amounts of endotoxin (LPS). These adverse effects were not observed if a lower (fourfold lower) dose of bacteria was given (Qadri *et al* 2005b, Savarino *et al,* to be published).

As is well known in the art, there are several types of CFs associated with human pathogenic strains of ETEC, but CFA/I, CFA/II and CFA/IV are the major types, currently associated with approximately 40-80% of clinical isolates. CFA/I is a single fimbrial antigen, whereas CFA/II and CFA/IV may be composed of more than one type of CF/CS proteins.

CF expression in wild-type ETEC appears to be restricted so that native strains only express a maximum of two or three types of CF antigens and then in certain combinations. Thus, native CFA/II ETEC strains generally express either CS1 together with CS3, CS2 with CS3 or CS3 alone. Similarly, native CFA/IV ETEC strains generally express CS4 with CS6, CS5 with CS6 or CS6 alone. However, e.g. CS1 and CS2 have not been found in the same wild type strain, and similarly CS4 and CS5 are not expressed together in naturally occurring strains. Furthermore, expression of CS4, CS5 or CS6 together with CS1 or CS2 or CS3 has not been described for wild type strains.

A minimum requirement that has been proposed for a vaccine against ETEC is that it should have the potential to induce protection against ETEC strains expressing CFA/I and the different subcomponents of CFA/II and CFA/IV. i.e. CS1-CS6. Thus, ETEC vaccines based on wild type strains may require a minimum of at least 5 bacterial strains, expressing CFA/I, CS1+CS3, CS2+CS3, CS4+CS6, and CS5+CS6.

One strategy to solve this problem was addressed in our pending International patent application PCT/SE2007/050051 where strains of *E*. *coli* which express elevated levels of ETEC CFs were used. The amount of these antigens can thus be increased in the vaccine without increasing the overall number of *E. coli* bacteria in the vaccine. This strategy was combined with insertion of at least one recombinant plasmid expressing an ETEC CF into a bacterial cell expressing another ETEC CF, thus providing an unnatural combination of expressed CFs from one bacterial strain.

The present application presents another solution to the problem of constructing an *E*. *coli* strain which provides expression of elevated numbers of ETEC fimbriae for use in vaccines against diarrhea.

### Description of the invention

The present invention provides, in one aspect a recombinant operon comprising a gene assembly wherein there are at least two structural genes coding for at least two major subunits of colonization factor antigens (CFs) associated with enterotoxigenic *Escherichia coli* bacteria (ETEC). It is thus possible to express from this operon at least two structural genes coding for at least two major subunits of CFs, which enables reduction of the number of bacterial cells needed in a vaccine composition.

In another aspect of the invention a host cell is genetically engineered to comprise the above mentioned recombinant operon wherein said operon is located on an episomal element or integrated in the chromosome of the said host cell.

In a first embodiment of the invention the episomal element in the host cell is a plasmid.

In a second embodiment of the invention the at least two major subunits of colonization factor antigens (CFs) are the same or different. If for example operon 1 of a CF comprises A, B, C and D, where B is the major subunit and operon 2 of another CF comprises A', B', C' and D', where B' is the major subunit, then operon 1 and 2 can be combined to A, B, B', C and D or A', B', B, C' and D'. In both cases two different major subunits will be expressed from the same operon. If the two major subunits of colonization factor are the same the operon would be e. g. A, B, B, C, D if B is the major subunit. This could induce a greater immune response against the major subunit B from a smaller amount of cells. An other example of the order of the gene fragments would be, B, A, C, D and the operon with two structural genes of major subunits would be B, B, A, C, D or B',B A,C,D, respectively. In case several structural genes coding for major subunits of CFs are included in the same operon, these would be named B", B"' etc. An example of this strategy is given below, where the structural gene coding for the major subunit of CS2 is included in the CFA/I operon resulting in expression of the hybrid fimbriae CFA/I+CS2.

In a third embodiment of the invention the CFs are selected from the group consisting of CFA/I, CS1, CS2, CS3, CS4, CS5, CS6, CS14, CS17, CS19 and putative colonization factor 071 (PCF071). Of these CFA/1, CS1, CS2, CS4, CS14, CS17, CS19 and putative colonization factor 071 (PCD071) have a similarly constructed operon. These are thus preferably combined with each other.

In a fourth embodiment of the invention the host cell expresses the at least two major subunits of CFs in the operon.

In a fifth embodiment of the invention the host cell is a viable microorganism selected from the group consisting of bacteria and unicellular eukaryotes. The bacteria may be of such species as *Vibrio cholerae* and *Escherichia coli* and the unicellular eukaryotes may be of such species as yeasts and in particular yeast species such as *Saccharomyces cerevisiae, Schizosaccharomyces pombe and Pichia pastoris.*

In a sixth embodiment of the invention the host cell is an *E. coli* cell.

In a seventh embodiment of the invention the host cell is a non-toxigenic *E. coli* cell.

In an eight embodiment of the invention the host cell does not express an antibiotic resistance gene.

In a ninth embodiment of the invention the host cell carries one or more complementable chromosomal deletions or mutations that are complemented by one or more plasmids. These chromosomal deletions may for instance be located where genes necessary for production of essential amino acids are located.

Another aspect of the invention concerns a method of producing a host cell carrying at least one recombinant plasmid capable of expressing at least two major subunits of colonization factor antigens (CFs) associated with enterotoxigenic *Escherichia coli* bacteria (ETEC), comprising the steps of assembling in an operon genes or gene fragments required for expression of a hybrid ETEC CF; a promoter that controls the expression of the subunits ; either integrating the operon into the genom of the host cell or transforming the host cell with a plasmid comprising the operon, a selection marker for plasmid maintenance and an origin of replication.

Host cells generated according to the invention can be used to manufacture a vaccine against ETEC diarrhea.

Thus, yet another aspect of the invention is directed to a vaccine against diarrhea comprising at least one host strain according to the invention together with pharmaceutically acceptable excipients, buffers and/or diluents, such as those selected for oral delivery of the vaccine. Suitable excipients, buffers and/or diluents for a vaccine can be found in the European or US pharmacopoeia.

In a final aspect of the invention there is provided the use of an operon according to the present invention in the production of a vaccine.

Since the described methods avoid the previous limitations of CF antigen expression in certain naturally occurring combinations, the invention may provide a vaccine against diarrhea comprising as few as 1-2 host strains which together express the major subunits of CFA/I, CS1, CS2, CS4, i.e. at least two major subunits of CFs by each strain. Thus, the vaccine will in total comprise of fewer strains, perhaps with the added advantage of being able to use lower doses of each strain than in earlier tested killed ETEC vaccines.

The expressed CFs contemplated for the purpose of vaccine production according to the invention are associated with ETEC causing intestinal infection and disease in mammals, especially humans.

Preferably the cells according to the invention express said CFs on the host cell surface.

The expression level obtained with the invention of CFs on the surface of host cells can be detected by an immunological method, e.g. by applying an inhibition ELISA assay.

In an embodiment of the invention, the major subunits of CFs that are expressed by a cell of the invention are expressed in a form that allow them to react with specific antibodies raised against corresponding major subunits of CFs from ETEC strains originally isolated from the stool of a mammal with intestinal ETEC infection.

In another embodiment of the invention, the CFs that are expressed by a cell of the invention are expressed in a form that when the cells are used in an effective amount for immunization of a mammal, leads to formation of antibodies against the expressed major subunits of CFs that can react with corresponding major subunits of CFs from ETEC strains originally isolated from the stool of a mammal with intestinal ETEC infection.

In yet another embodiment of the invention, the hybrid CFs that are expressed by a cell of the invention are expressed in a form that after inactivation of the cell by formalin treatment or other means, allows them to react with specific antibodies raised against corresponding subunits of CFs from ETEC strains originally isolated from the stool of a mammal with intestinal ETEC infection.

In still another embodiment of the invention, the CFs that are expressed by a cell of the invention are expressed in a form that after inactivation of the cell by formalin treatment or other means, when the cell is used in an effective amount for immunization of a mammal, leads to formation of antibodies against the expressed CFs that can react with corresponding CFs from ETEC strains originally isolated from the stool of a mammal with intestinal ETEC infection.

Host cells according to the invention are cultured by methods for *in vitro* culturing of the cells in liquid media providing expression of said hybrid CFs.

A cultured cell of the invention may be inactivated by using mild treatment with formalin or phenol or other means, thereby preventing the cell from replication, and resulting in a cell that retains the expressed hybrid CFs in essentially the same amounts (at least 50% of the original amount), and with essentially the same reactivity with antibodies and almost the same immunogenicity as for the cell before the inactivation.

One or several of the host cells of the invention is (are) especially suitable for use in a method of vaccinating a mammal against diarrhea, which comprises administering to the mammal a strain or combination of cells according to the invention.

In an embodiment of the invention, one or several of the host cells of the invention is (are) used alone or in combination as a vaccine, for vaccination of a mammal, such as a piglet, a calf, a lamb or a horse, or in particular a human being. Such a vaccine is preferably administered by the oral route.

The invention will now be illustrated by the following description of the drawing, the drawing, the sequence listing, the Materials and Methods and the Examples as well as the Table, but it should be understood that the invention is not limited to any disclosed details.

### Description of the drawing

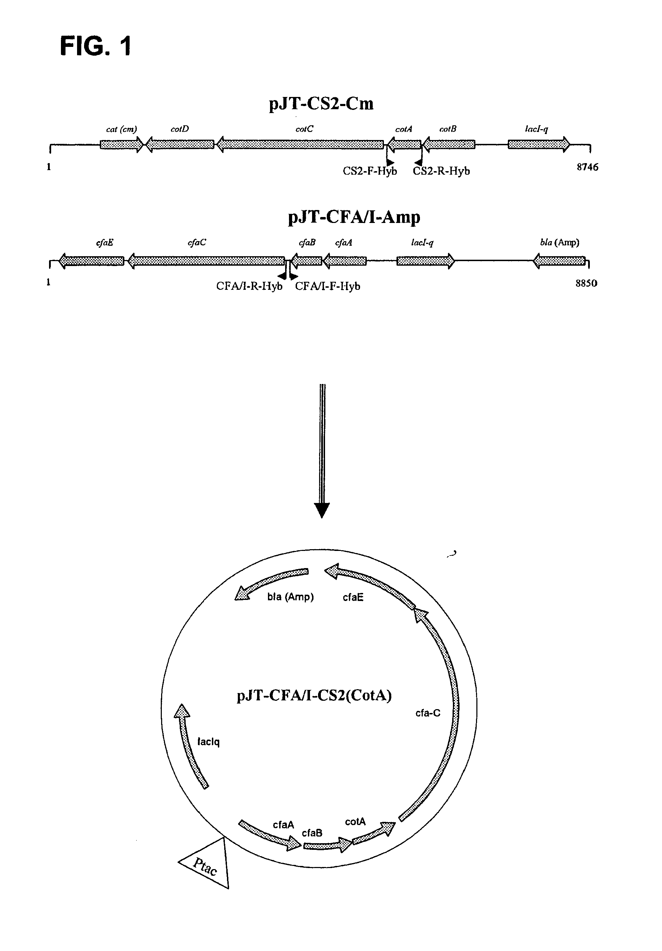

Fig.1 The figure shows construction of the hybrid expression vector pJT-CFA/I-CS2(CotA). Using specific primers to amplify the CotA and the entire pJT-CFA/I-Amp, two fragments were amplified followed by ligation.

### Materials and methods

### Bacterial strains and culture conditions

Strains described in this application are listed in Table 1. Construction of recombinant cells expressing a hybrid protein comprising two major subunits of members of a fimbriae family is exemplified by construction of a strain expressing the major subunits of both CFA/I and CS2.

Strains were kept frozen at -70°C in a glycerol-containing freezing medium until used. After inoculation of an agar plate at 37° C over night to ascertain growth and purity bacteria were grown in CFA broth (Casamino acids 10g, Yeast extracts 1.5g, MgSO₄7H₂O 102 mg, MnCl₂4H₂O 8 mg per liter), at 37°C with shaking for 16-18 h. When necessary, the medium was supplemented with chloramphenicol (12.5 µg/ml) or ampicillin (100 µg/ml).

### Cloning of ETEC CFs operon in expression vectors

### Production of CFA/I+CS2 as hybrid fimbriae

Production of a hybrid fimbriae that consists of the major subunit of CS2, and the usher, chaperon and the minor and major subunits of CFA/I, was done in several steps. A fragment containing the major subunit of CS2, CotA, was amplified by PCR using the Expand High Fidelity PCR System (Roche Diagnostics GmbH) the primers CS2-F-Hyb and CS2-R-Hyb (Table 1) and using the plasmid pJT-CS2-Cm (see pending patent application PCT/SE2007/050051) as template. Additionally, the plasmid pJT-CFA/I-Amp (See pending patent application PCT/SE2007/050051) was subjected to reverse PCR, using the primers CFA/I-F-Hyb and CFA/I-R-Hyb (Table 1), resulting in a fragment containing the original plasmid. Following restriction of both fragments with Eco31I, both fragments were ligated, resulting in a plasmid containing the entire operon of CFA/I and CotA downstream the CfaB.

### Expression of CFs

An over night culture of each recombinant TOP10 strain was diluted 1/100 in CFA broth, supplemented, with 100 or 12.5 µg/ml of ampicillin or chloramphenicol, respectively (Table 1), and incubated for 2 h at 37°C and 150 rev/min, followed by addition of IPTG to the final concentration of 1 mM and incubation with the same conditions for additional 4 h. The bacteria were then harvested and re-suspended in PBS.

### Dot blot test

Specific monoclonal anti-CFAs antibodies were used to evaluate the expression of each CFAs on the cloned strains, as described previously (Binsztein *et al* 1991). Briefly, 2 µl of bacterial culture (10⁹ bacteria/ml in PBS) that have been washed with PBS, and induced with IPTG for expression CFA/I, were applied on the nitrocellulose filter papers and incubated with the MAbs followed by goat anti-mouse IgG, conjugated with HRP, for 1.5 h each. The final development was performed by 4-chloro-1-naphtol-H₂O₂ in TBS for up to 15 min.

### Hemagglutination

Fresh human or bovine erythrocytes were washed twice in 0.85% NaCl, suspended to 3% in saline with 1 % D-mannose. Ten µl of this mixture, and the same volume of the tested bacterial suspension in PBS (10⁹ bacteria/ml) which were inducted for expression of the CFAs and washed with PBS, mixed and the hemagglutination was observed in 2 min at room temperature.

### Electron Microscopy

Ten µl of each bacterial suspension (10¹⁰ bacteria/ml in PBS), that had been washed once with PBS, were applied on parafilm. Formvar- coated grids were put on the suspension for 2 min. The grids were then washed twice, 10 sec each, by applying them on 25 µl of PBS-1% BSA on parafilm, followed by incubating the grids for 15 min with 25 µl specific monoclonal antibody diluted in PBS-Tween 0.05%-BSA 0.1%. The grids were washed 6 times with PBS-1% BSA, as above, and then incubated for 15 min with anti-mouse IgG-gold conjugate (Amersham International, Amersham, UK) in PBS-0.1%BSA-0.05% Tween. The grids were then washed three times with PBS-0.1%BSA, and three times with distilled water. Negative staining was performed by applying the grids on 25 µl of 1% ammonium molybdate (pH 7.0) for 50-60 sec, followed by air-drying the grids on a filter paper for 5 min. The grids were stored at 4°C until examined by electron microscopy.

### ELISA

The amount of CFA/I or CS2 on the bacterial surface was quantified by an inhibition ELISA assay (Lopez-Vidal *et al* 1988), and the titers of IgA or IgG+M antibodies in serum determined by ELISA assay, as described previously (Rudin *et al,* 1994).

### Inactivation of bacteria by formalin

To kill the bacteria, the culture of each strain was washed and re-suspended with PBS to a density of 10¹⁰ bacteria/ml in PBS. Formalin was added to a final concentration of 0.1 M, and the suspension incubated for 2h at 37°C and agitated with 60 rpm, followed by incubation of the suspensions at 4°C for 3 days. The bacteria were then washed, re-suspended with the same volume of PBS, and 100µl of the bacterial suspension was spread onto blood agar and incubated at 37°C for up to a week to check for growth.

### Mice immunization

Female Balb/c mice (6-8 weeks of age) were used for the immunizations in oral route. Cultures of induced and formalin killed reference strains 325542-3 and 58R957, and the recombinant strain TOP10-CFA/I-CS2, were washed and re-suspended in PBS to the desired bacterial density. 10⁹ bacteria together with 10 µg CT were used for immunization by the oral route, as previously described (Rhagavan et al, 200). All mice were given two identical immunizations two weeks apart, and bleedings were collected immediately before the first dose and two weeks after the second dose.

### Statistical analysis

All ELISA and inhibition ELISA experiments were performed in duplicates and repeated at least three times on different days. Dot blot experiments for each particular test were repeated at least twice. Statistical analyses were conducted by the student's t-test and P<0.05 was regarded significant.

### Examples

### Example 1.

**Production of hybrid fimbriae:** We examined the possibility of expressing a hybrid fimbriae consisting of the major subunits of both CFA/I and CS2. Construction of an expression vector expressing such hybrid fimbriae is described in materials and methods, and depicted in Fig.1. The vector was then propagated in Top10 strain, resulting in a recombinant strain expressing a fimbriae consisting of both major subunits of CFA/I and CS2. The expression was verified by using Transmission Electron Microscopy (TEM) and specific MAbs against each of major subunits, i.e. α-CFA/I MAb (1:6) ~ goat α-mouse IgG 20 mn gold and Biotinylated α-CS2 MAb (10:3) ∼ Streptavidin 10 nm gold.

**Table 1 - List of strains, plasmids, and primers used in this study.**

| Strains, plasmid and primers | Relevant characteristic | Source |
|---|---|---|
| | | |
| Strains: | | |
| *E. coli* TOP10 | K12, F⁻ lambda⁻ | Invitrogen |
| TOP10-CFA/I- | TOP10 expressing CFA/I and CS2(cotA) | This study |
| CS2(cotA)-Amp | | |
| ETEC 325542-3 | CFA/I ref. strain | |
| ETEC 278485-2 | CS2 ref. strain | |
| Plasmid: | | |
| pJT-CFA/I-Amp | 8850 bp; amp^{r} | |
| pJT-CS2-Cm | 8472 bp; cm^{r} | |
| pJT-CFA/I-CS2(cotA)- | | |
| Amp | | |
| Primers: | | |
| CFA/I-F | | |
| SEQ ID NO:1 | 5'-CGGTCTCGAATTCTGATGGAAGCTCAGGAGG | Acc. no. M55661 |
| CFA/I-R | | |
| SEQ ID NO:2 | 5'-CGGTCTCAAGCTTTCTAGAGTGTTTGACTACTTGG | |
| CS2-F | | |
| SEQ ID NO:3 | 5'-CGGTCTCGAATTCTTCTTGAAAGCCTCATGC | Acc. no. Z47800 |
| CS2-R | | |
| SEQ ID NO:4 | 5'-CGGTCTCAAGCTTTTTACAGACTTGAACTACTAGG | |
| CFA/I-F-Hyb | | |
| SEQ ID NO:5 | 5'-CGGTCTCTGCATTAAAGAATCAGGATCCCAAAGTC | |
| CFA/I-R-Hyb | | |
| SEQ ID NO:6 | 5'-CGGTCTCTCATCTGGTATGTTTATACATCCCTC | |
| CS2-F-Hyb | | |
| SEQ ID NO:7 | 5'-CGGTCTCTGATGTTTCTTTAATAACAGGGTGAC | |
| CS2-R-Hyb | | |
| SEQ ID NO:8 | 5'-CGGTCTCTATGCTCAATAACCACTGTATAAGGG | |

### References:

Ahren, C. M. Jertborn, and A.M. Svennerholm. 1998. Intestinal immune responses to an inactivated oral enterotoxigenic Escherichia coli vaccine and associated immunoglobulin A responses in blood. Infect. Immun. 66:3311-3316.
Binsztein, N., M.J. Jouve, G.I. Viboud, L.L. Moral, M. Rivas, I. Orskov, C. Ahrén, and A-M. Svennerholm. 1991. Colonization factors of enterotoxigenic Escherichia coli isolated from children with diarrhea in Argentina. J. Clin. Microbiol. 29:1893-1898.
Gaastra, W., and A.M. Svennerholm. 1996. Colonization factors of human enterotoxigenic Escherichia coli (ETEC). Trends Microbiol. 4:444-452.
Jertborn, M., C. Ahren, J. Holmgren, A.M. Svennerholm. 1993. Safety and immunogenicity of an oral inactivated enterotoxigenic Escherichia coli vaccine. Vaccine 16: 255-60.
Levine, M.M., J.A. Giron, and F. Noriega. 1994. Fimbrial vaccines,. In Fimbriae: adhesion, biogenics, genetics and vaccines, P. Klemm (ed.), pp.255-270. CRC Press, Boca Raton, Fla.
Qadri F., A.M. Svennerholm, A.S.G. Faruque, and Sack R.B. 2005a. Enterotoxigenic Escherichia coli in developing countries: epidemiology, microbiology, clinical features, treatment, and prevention. Clin. Microbiol. Rev. 18:465-483.
Qadri, F., T. Ahmed, F. Ahmed, Y.A. Begum, D.A. Sack, A.M. Svennerholm and the PTE Study Group. 2005b. Reduced doses of oral killed enterotoxigenic Escherichia coli plus cholera toxin B subunit vaccine is safe and immunogenic in Bangladeshi infants 6-17 months of age: Dosing studies in different age groups. Vaccine 24:1726-1733.
Rhagavan S, M. Hjulström, J. Holmgren and A.M.Svennerholm. 2002. Protection against experimental Helicobacter pylori infection after immunization with inactivated H. pylori whole cell vaccines. Infect. Immun. 70:6383-6388.
Rudin, A., M.M. McConnell, A.M. Svennerholm. 1994. Monoclonal antibodies against enterotoxigenic Escherichia coli colonization factor antigen I (CFA/I) that cross-react immunologically with heterologous CFAs. Infect. Immun. 62:4339-4346.
Sack, D.A., J. Shimko, O. Torres et al. 2002. Safety and efficacy of a killed oral vaccine for enterotoxigenic E. coli diarrhea in adult travelers to Guatemala and Mexico. 42nd Interscience Conference on Antimicrobial Agents and Chemotherapy. San Diego, CA. Abstract.
Sánchez J., and J. Holmgren. 2005. Virulence factors, pathogenesis and vaccine protection in cholera and ETEC diarrhea. Curr. Opin. Immunol. 17:388-98.
Savarino, S.J., F.M. Brown, E. Hall, S. Bassily, F. Youssef, T. Wierzba, L. Peruski, N.A. El-Masry, M. Safwat, M. Rao, M. Jertborn, A.M. Svennerholm, Y.J. Lee, and J.D. Clemens. 1998. Safety and immunogenicity of an oral, killed enterotoxigenic Escherichia coli-cholera toxin B subunit vaccine in Egyptian adults. J. Infect. Dis. 177:796-799.
Savarino, S.J., E. Hall,. S. Bassily,. F.M. Brown, F. Youssef, T.F. Wierzba,. L. Peruski, N.A. El-Masry, M. Safwat, M. Rao, H. El Mohamady, R. Abu-Elyazeed, A.Naficy, A.M. Svennerholm, M. Jertborn, Y.J. Lee, and J.D. Clemens. 1999. Oral, inactivated, whole cell enterotoxigenic Escherichia coli plus cholera toxin B subunit vaccine: results of the initial evaluation in children.. J. Infect. Dis. 179:107-14.
Svennerholm A.-M., Y.L. Vidal, J. Holmgren, M.M. McConnell, and B. Rowe. 1988. Role of PCF8775 antigen and its coli surface subcomponents for colonization, disease, and protective immunogenicity of enterotoxigenic Escherichia coli in rabbits. Infect. Immun. 56:523-528.
Svennerholm, A.-M., C. Wenneras, J. Holmgren, M. M. McConnell, and B. Rowe. 1990. Roles of different coli surface antigens of colonization factor antigen II in colonization by and protective immunogenicity of enterotoxigenic Escherichia coli in rabbits. Infect. Immun. 58:341-346.
Svennerholm, A.-M., and J. Holmgren. 1995. Oral vaccines against cholera and enterotoxigenic Escherichia coli diarrhea. Adv. Exp. Med. Biol. 371 B:1623-1628.
Svennerholm, A.-M., and S. J. Savarino. 2004. Oral inactivated whole cell B subunit combination vaccine against enterotoxigenic Escherichia coli. In New Generation Vaccines : 3rd ed. Eds: Levine M.M. et al., pp. 737-750, Marcel Decker, New York, NY.
Svennerholm, A.-M., and D. Steele. 2004. Progress in enteric vaccine development. In: Microbial-gut interactions in health and disease, Ed. M. Farthing. Best Pract. Res. Clin. Gastroenterol. 18: 421-445.

## Claims

1. A recombinant operon comprising a gene assembly wherein there are at least two structural genes coding for at least two major subunits of colonization factor antigens (CFs) associated with enterotoxigenic *Escherichia coli* bacteria (ETEC).

2. A host cell genetically engineered to comprise a recombinant operon according to claim 1, wherein said operon is located on an episomal element or integrated in the chromosome of said host cell.

3. A host cell according to claim 2, wherein the episomal element is a plasmid.

4. The host cell according to claim 2 or 3, wherein the at least two major subunits of colonization factor antigens (CFs) are the same or different.

5. The host cell according to any one of claims 2 - 4, wherein CFs are selected from the group consisting of CFA/I, CS1, CS2, CS4, CS14, CS17, CS19 and putative colonization factor 071 (PCFO71)

6. The host cell according to any one of claims 2-5, wherein said cell expresses the at least two major subunits of CFs.

7. The host cell according to any one of claims 2-6 wherein the host cell is a viable microorganism selected from the group consisting of bacteria and unicellular eukaryotes.

8. The host cell according to claim 7, wherein the host cell is an *Escherichia coli* cell.

9. The host cell according to claim 8, wherein said *E. coli* cell is a non-toxigenic *E*. *coli* cell.

10. The host cell according to any one of claims 2-9, wherein said cell does not express an antibiotic resistance gene.

11. The host cell according to any one of claims 2-10, wherein said host cell carries one or more complementable chromosomal deletion(s) or mutation(s) that are complemented by one or more plasmid(s).

12. A method of producing a host cell capable of expressing at least two major subunits of colonization factor antigens (CFs) associated with enterotoxigenic *Escherichia coli* bacteria (ETEC), comprising the steps of
assembling in an operon genes or gene fragments required for expression of a hybrid ETEC CF; a ETEC promoter that controls the expression of the subunits; either integrating the operon into the genome of the host cell or transforming the host cell with a plasmid comprising the operon, a selection marker for plasmid maintenance and an origin of replication.

13. A vaccine composition against diarrhea comprising at least one host cell according to any one of claims 2-11, together with pharmaceutically acceptable excipients, buffers, and/or diluents.

14. The vaccine according to claim 13, wherein the pharmaceutically acceptable excipients, buffers, and/or diluents are selected for oral delivery of the vaccine.

15. Use of an operon according to claim 1 in the production of a vaccine.

## Patentansprüche

1. Rekombinantes Operon, umfassend eine Genanordnung, wobei wenigstens zwei Strukturgene vorliegen, die für wenigstens zwei Hauptuntereinheiten von mit enterotoxigenen Escherichia coli-Bakterien (ETEC) assoziierten Kolonisationsfaktorantigenen (CFs) codieren.

2. Wirtszelle, gentechnisch hergestellt, so dass sie ein rekombinantes Operon nach Anspruch 1 umfasst, wobei das Operon auf einem episomalen Element liegt oder im Chromosom der Wirtszelle integriert ist.

3. Wirtszelle nach Anspruch 2, wobei es sich bei dem episomalen Element um ein Plasmid handelt.

4. Wirtszelle nach Anspruch 2 oder 3, wobei die wenigstens zwei Hauptuntereinheiten von Kolonisationsfaktorantigenen (CFs) gleich oder verschieden sind.

5. Wirtszelle nach einem der Ansprüche 2 - 4, wobei CFs ausgewählt sind aus der Gruppe bestehend aus CFA/I, CS1, CS2, CS4, CS14, CS17, CS19 und putativem Kolonisationsfaktor 071 (PCFO71).

6. Wirtszelle nach einem der Ansprüche 2 - 5, wobei die Zelle die wenigstens zwei Hauptuntereinheiten von CFs exprimiert.

7. Wirtszelle nach einem der Ansprüche 2-6, bei der es sich um einen aus der Gruppe bestehend aus Bakterien und einzelligen Eukaryonten ausgewählten lebensfähigen Mikroorganismus handelt.

8. Wirtszelle nach Anspruch 7, bei der es sich um eine Escherichia coli-Zelle handelt.

9. Wirtszelle nach Anspruch 8, wobei es sich bei der E. coli-Zelle um eine nicht toxigene E. coli-Zelle handelt.

10. Wirtszelle nach einem der Ansprüche 2 - 9, wobei die Zelle kein Antibiotikaresistenzgen exprimiert.

11. Wirtszelle nach einem der Ansprüche 2 - 10, wobei die Wirtszelle eine oder mehrere komplementierbare chromosomale Deletion(en) oder Mutation(en) trägt, die durch ein oder mehrere Plasmid(e) komplementiert werden.

12. Verfahren zur Herstellung einer Wirtszelle, die in der Lage ist, wenigstens zwei größere Untereinheiten von mit enterotoxigenen Escherichia coli-Bakterien (ETEC) assoziierten Kolonisationsfaktorantigenen (CFs) zu exprimieren, wobei man in den Verfahrensschritten
in einem Operon für die Expression eines Hybrid-ETEC-CF benötigte Gene oder Genfragmente, einen ETEC-Promotor, der die Expression der Untereinheiten kontrolliert, zusammenstellt, entweder das Operon in das Genom der Wirtszelle integriert oder die Wirtszelle mit einem das Operon, einen Selektionsmarker zur Plasmiderhaltung und einen Replikationsursprung umfassenden Plasmid transformiert.

13. Impfstoffzusammensetzung gegen Diarrhö, umfassend wenigstens eine Wirtszelle nach einem der Ansprüche 2 - 11, zusammen mit pharmazeutisch unbedenklichen Hilfsstoffen, Puffern und/oder Verdünnungsmitteln.

14. Impfstoff nach Anspruch 13, wobei die pharmazeutisch unbedenklichen Hilfsstoffe, Puffer und/oder Verdünnungsmittel zur oralen Zuführung des Impfstoffs ausgewählt sind.

15. Verwendung eines Operon nach Anspruch 1 bei der Herstellung eines Impfstoffs.

## Revendications

1. Opéron recombinant comprenant un assemblage génétique comprenant au moins deux gènes structuraux codant pour au moins deux sous-unités majeures d'antigènes de facteur de colonisation (CF) associés à des bactéries *Escherichia coli* entérotixinogènes (ETEC).

2. Cellule hôte génétiquement modifiée pour comprendre un opéron recombinant selon la revendication 1 où ledit opéron est situé sur un élément épisomique ou intégré dans le chromosome de ladite cellule hôte.

3. Cellule hôte selon la revendication 2, dans laquelle l'élément épisomique est un plasmide.

4. Cellule hôte selon la revendication 2 ou 3, dans laquelle les au moins deux sous-unités majeures d'antigènes de facteur de colonisation (CF) sont identiques ou différentes.

5. Cellule hôte selon l'une quelconque des revendications 2 à 4, où les CF sont choisis dans le groupe constitué de CFA/I, CS1, CS2, CS4, CS14, CS17, CS19 et le facteur de colonisation putatif 071 (PCF071).

6. Cellule hôte selon l'une quelconque des revendications 2 à 5, où ladite cellule exprime les au moins deux sous-unités majeures de CF.

7. Cellule hôte selon l'une quelconque des revendications 2 à 6 où la cellule hôte est un micro-organisme viable choisi dans le groupe constitué de bactéries et d'eucaryotes unicellulaires.

8. Cellule hôte selon la revendication 7, où la cellule hôte est une cellule d' *Escherichia coli*.

9. Cellule hôte selon la revendication 8, où ladite cellule *E. coli* est une cellule *E. coli* non toxinogène.

10. Cellule hôte selon l'une quelconque des revendications 2 à 9, où ladite cellule n'exprime pas un gène de résistance aux antibiotiques.

11. Cellule hôte selon l'une quelconque des revendications 2 à 10, où ladite cellule hôte comporte une ou plusieurs délétion(s) ou mutation(s) chromosomiques complémentables qui sont complémentées par un ou plusieurs plasmide(s).

12. Procédé de production d'une cellule hôte capable d'exprimer au moins deux sous-unités majeures d'antigènes de facteur de colonisation (CF) associés à des bactéries *Escherichia coli* entérotixinogènes (ETEC), comprenant les étapes consistant à assembler dans un opéron des gènes ou des fragments de gène requis pour l'expression d'un CF ETEC hybride ; un promoteur ETEC qui contrôle l'expression des sous-unités ; l'intégration de l'opéron dans le génome de la cellule hôte ou la transformation de la cellule hôte avec un plasmide comprenant l'opéron, un marqueur de sélection pour la maintenance de plasmide et une origine de réplication.

13. Composition de vaccin contre la diarrhée comprenant au moins une cellule hôte selon l'une quelconque des revendications 2 à 11, conjointement avec des excipients, tampons et/ou diluants pharmaceutiquement acceptables.

14. Vaccin selon la revendication 13, dans lequel les excipients, tampons, et/ou diluants sont choisis pour administration orale du vaccin.

15. Utilisation d'un opéron selon la revendication 1 dans la production d'un vaccin.


### SEQUENCE LISTING

<110> SBL Vaccin AB
<120> Hybrid operon for expression of colonization factor (CF) antigens of enterotoxigenic Escherichia coli.
<130> P40701283EP00
<160> 8
<170> PatentIn version 3.4
<210> 1
   <211> 31
   <212> DNA
   <213> primer CFA/I-F
<400> 1
   cggtctcgaa ttctgatgga agctcaggag g 31
<210> 2
   <211> 35
   <212> DNA
   <213> primer CFA/I-R
<400> 2
   cggtctcaag ctttctagag tgtttgacta cttgg 35
<210> 3
   <211> 31
   <212> DNA
   <213> primer CS2-F
<400> 3
   cggtctcgaa ttcttcttga aagcctcatg c 31
<210> 4
   <211> 35
   <212> DNA
   <213> primer CS2-R
<400> 4
   cggtctcaag ctttttacag acttgaacta ctagg 35
<210> 5
   <211> 35
   <212> DNA
   <213> primer CFA/I-F-Hyb
<400> 5
   cggtctctgc attaaagaat caggatccca aagtc 35
<210> 6
   <211> 33
   <212> DNA
   <213> primer CFA/I-R-Hyb
<400> 6
   cggtctctca tctggtatgt ttatacatcc ctc 33
<210> 7
   <211> 33
   <212> DNA
   <213> primer CS2-F-Hyb
<400> 7
   cggtctctga tgtttcttta ataacagggt gac 33
<210> 8
   <211> 33
   <212> DNA
   <213> primer CS2-R-Hyb
<400> 8
   cggtctctat gctcaataac cactgtataa ggg 33